(19) Europäisches Patentamt
European Patent Office
Office européen des brevets

(11) **EP 0 523 482 B1**

(12) **EUROPÄISCHE PATENTSCHRIFT**

(45) Veröffentlichungstag und Bekanntmachung des Hinweises auf die Patenterteilung:
**29.10.1997 Patentblatt 1997/44**

(51) Int. Cl.$^6$: **C07C 5/05**, C07C 11/08

(21) Anmeldenummer: **92111353.6**

(22) Anmeldetag: **03.07.1992**

(54) **Verfahren zur selektiven Hydrierung von butadienreichen Roh-C4-Schnitten**

Method for the selective hydrogenation of crude C4-cuts rich in butadiene

Procédé pour l'hydrogénation sélective de coupes-C4 brutes, riches en butadiène

(84) Benannte Vertragsstaaten:
**BE DE ES FR GB IT NL**

(30) Priorität: **13.07.1991 DE 4123246**

(43) Veröffentlichungstag der Anmeldung:
**20.01.1993 Patentblatt 1993/03**

(73) Patentinhaber: **BASF Aktiengesellschaft**
**67063 Ludwigshafen (DE)**

(72) Erfinder:
• **Polanek, Peter, Dr.**
**W-6940 Weinheim (DE)**
• **Posselt, Dietmar, Dr.**
**W-6700 Ludwigshafen (DE)**
• **Schreyer, Peter, Dr.**
**W-6940 Weinheim (DE)**

(56) Entgegenhaltungen:
EP-A- 0 288 362     DD-A-  50 823
DE-B- 1 568 542     DE-C-  740 478
US-A- 3 478 123

• CHEMICAL ABSTRACTS, vol. 88, no. 1, 2. Januar 1978, Columbus, Ohio, US; abstract no. 5972v, P. KRIPYLO ET AL.
• COMPTES RENDUS DES SEANCES DE L'ACADEMIE DES SCIENCES. SERIE II:MECANIQUE, PHYSIQUE, CHIMIE, SCIENCES DE LA TERRE, SCIENCES DE L'UNIVERS Bd. 311, Nr. II, 1990, MONTREUIL, FR, Seiten 313 - 317, M. MEYER et al: " Hydrogénation du butadiène sur Palladium, influence de l'addition d'Argent"
• PATENT ABSTRACTS OF JAPAN vol. 11, no. 394 (C-465)(2841) 23. Dezember 1987; & JP-A-62 153 230
• PATENT ABSTRACTS OF JAPAN vol. 10, no. 156 (C-351)(2212) 5. Juni 1986; & JP-A-61 11 145
• DATABASE WPI Week 7528, Derwent Publications Ltd., London, GB; AN 75-46972W; & JP-A-50 016 325
• CHEMICAL ABSTRACTS, vol. 82, no. 21, 26. Mai 1975, Columbus, Ohio, US; abstract no. 138918e, D.A. BUCHANAN ET AL.

**Beschreibung**

Die Erfindung betrifft ein Verfahren zur selektiven Hydrierung von Butadien in butadienreichen Roh-$C_4$-Strömen zu Butenen. Solche $C_4$-Kohlenwasserstoffgemische entstehen hauptsächlich beim Steamcracken von mineralölstämmigen Kohlenwasserstoffen, z.B. Naphtha. Neben der Hauptkomponente 1,3-Butadien können diese Kohlenwasserstoffgemische auch geringe Mengen an Verbindungen mit kumulierten Doppelbindungen und/oder acetylenischen Dreifachbindungen enthalten, die beim vorliegenden Verfahren ebenfalls selektiv zu den Butenen hydriert werden.

Die Zusammensetzung des Roh-$C_4$-Schnitts aus dem Steamcracker kann in weiten Bereichen variieren (s. Tabelle 1).

Tabelle 1

| Zwei typische Zusammensetzungen von $C_4$-Schnitten eines Steamcrackers angegeben in Gewichtsprozenten | | |
|---|---|---|
| | I | II |
| Butadien | 46 | 63 |
| Buten-1 | 13 | 9 |
| trans-Buten-2 | 4 | 3 |
| cis-Buten-2 | 3 | 2 |
| i-Buten | 24 | 20 |
| i-Butan | 3 | 0,5 |
| n-Butan | 6 | 2 |
| Vinylacetylen | 1 | 0,5 |

Die Zusammensetzung ist im wesentlichen abhängig von dem Einsatzstoff und den Spaltbedingungen des Steamcrackers. Üblicherweise ist in dem Roh-$C_4$-Schnitt zwischen 40 bis 50 Gew.-% Butadien enthalten.

Grundsätzlich können mit dem erfindungsgemäßen Verfahren alle, wie auch immer gewonnenen $C_4$-Schnitte mit Butadiengehalten bis 80 Gew.-% selektiv hydriert werden.

Die weitere Verarbeitung solcher Roh-$C_4$-Schnitte erfolgte bisher üblicherweise in der Weise, daß das Butadien durch Extraktionsverfahren mit geeigneten Lösungsmitteln abgetrennt wurde. Solche Verfahren sind beispielsweise in DE 2 724 365 ausführlich beschrieben und in zahlreichen technischen Anlagen realisiert. Das so isolierte Butadien kann z.B. zu Styrol-Butadien-Copolymerisaten, die u.a. als Verpackungen für Lebensmittel Verwendung finden, weiterverarbeitet werden.

Falls eine Butadienabtrennung ökonomisch nicht sinnvoll ist, bietet sich als alternative Verarbeitung die selektive Hydrierung des Butadiens zu den Butenen an. Die Butene stehen dann als Wertprodukt zur Weiterverarbeitung zu höherveredelten Chemikalien zur Verfügung.

Mögliche Verarbeitungswege für Butene sind z.B.:

-    Umsetzung von Isobuten zu Methyl-tert.-butylether oder tert.-Butylalkohol

-    Isolierung von Isobuten zur Herstellung von Polyisobuten bzw. anderen Folgeprodukten

-    Isolierung von Buten-1, z.B. durch Feindestillation

-    Dimerisierung der n-Butene zu Okten und Weiterverarbeitung zu Weichmacheralkoholen

Grundvoraussetzung für die beispielhaft genannten Veredelungsstufen ist ein nahezu butadienfreies Einsatzprodukt mit maximalen Butadiengehalten von ca. 0,2 Gew.-%, da sonst das Ausmaß der Nebenproduktenbildung durch unerwünschte Oligomeren- und Polymerbildung zu groß wird.

Eine weitere Verwendung ist beispielsweise die Copolymerisation von Ethylen mit Buten-1, wobei man sogenanntes lineares Nieder- und Hochdruckpolyethylen erhält, das aufgrund der im Vergleich zu üblichen Polyethylenen günstigeren anwendungstechnischen Eigenschaften in großen Mengen hergestellt wird.

2

Bisher wurden solche butadienfreien $C_4$-Schnitte ausschließlich über den Verfahrensschritt der Butadienextraktion erhalten. Hierdurch wird der Butadiengehalt auf ca. 1 Gew.-% abgesenkt.

Eine übliche Methode zur Entfernung von restlichen Butadienmengen aus bereits butenreichen $C_4$-Gemischen ist die selektive katalytische Hydrierung. So ist in DE-A 3 301 164 ein Verfahren zur Herstellung von butadienfreiem oder nahezu butadienfreiem Buten-1 aus $C_4$-Strömen mit bis zu 5 Gew.-% Butadiengehalt durch selektive Hydrierung des Butadiens beschrieben. Eine praktisch 100 %ige Selektivität auch bei Hydrierung auf sehr niedrige Restbutadienmengen von 10 ppm wird durch Zusatz von geringen Mengen CO erzielt. Gleichzeitig wird hierdurch die Isomerisierung von Buten-1 zu Buten-2 stark gehemmt bzw. verhindert.

Die selektive Resthydrierung von Butadien unter CO-Zusatz kann in an sich bekannter Weise durchgeführt werden, beispielsweise in der Gasphase, Flüssigphase oder Rieselphase. Vorzugsweise führt man die selektive Resthydrierung des Butadiens in der Flüssig- oder Rieselphase mit fest angeordneten Hydrierkatalysatoren durch.

In EP-A 0 087 980 wird die Durchführung der Reaktion in der Gasphase unter Einspeisung des Wasserstoffs an mindestens 2 Stellen im Reaktor beschrieben.

EP-A 288 362 lehrt ein Verfahren zur Isomerisierung von 1-Butenen in 2-Butene in Gegenwart von Wasserstoff. Der Einsatzstoff kann dabei Butadien in einer Menge von ca. 0,5 Gew.-% enthalten, dieser Anteil wird im Verfahren hydriert.

DE-A 15 68 542 lehrt ein Verfahren zur Isomerisierung von 1-Butenen in 2-Butene, wobei der Einsatzstoff 0,1 bis 10 Vol.-% Butadien enthalten kann. Dieser Anteil wird im Verfahren hydriert.

Aus DE 31 43 647 ist ferner ein Verfahren zur selektiven Hydrierung von $C_3$- und $C_4$-Kohlenwasserstoffen mit konjugierten und kumulierten Doppelbindungen und/oder acetylenischen Dreifachbindungen an fest angeordneten Trägerkatalysatoren bekannt, wobei a) der Wasserstoff homogen in feinverteilter Form in dem zu hydrierenden Kohlenwasserstoff vor Eintritt in den Reaktor gelöst wird und b) ein CO-Zusatz von mindestens 0,05 Massen-ppm notwendig ist. Hierdurch gelingt die selektive Hydrierung von butadienhaltigen Schnitten mit Butadiengehalten von bis zu 20 Gew.-%.

Diese Hydrierungen können mittels bekannter fest angeordneter Katalysatoren durchgeführt werden. Besonders geeignet sind Metalle aus der achten Nebengruppe und der ersten Nebengruppe des Periodensystems, die z.B. auf Aluminiumoxid, Bimsstein, Tone oder Silikate als Trägermaterial aufgebracht werden. Auch $TiO_2$ wurde gemäß EP-A 0 314 020 als Trägermaterial vorgeschlagen.

Geeignete Katalysatoren, die hauptsächlich zur selektiven Hydrierung von Butadien, Pentadien und Cyclopentadien verwendet werden, sind beispielsweise aus DE-A 32 07 029, DE-A 32 07 030, DE-A 31 43 647 und EP-A 00 11 906 bekannt. Es handelt sich hierbei um Palladium-Trägerkatalysatoren, wobei als Träger vor allem $Al_2O_3$ oder $SiO_2$ dienen.

Eine deutliche Verbesserung der Selektivität bei der Dien- oder Acetylenhydrierung läßt sich durch partielle Vergiftung, z.B. durch Promotierung der Pd-Katalysatoren mit Zn, Cd, Sn, Pb und Hg (s. G.C. Bond "Catalysis by Metals", Academic Press, London 1962, S. 99 und 297) erreichen. In neuerer Zeit vorgeschlagene Promotoren sind z.B. Ag (DE-A 31 19 850) oder mit anorganischen Basen vorbehandelte (DE-A 28 49 026) bzw. mit Alkali- und Erdalkalioxiden dotierte Katalysatoren (R.J. Peterson, Hydrogenation Catalysts, Noyes Data Corp., New York 1977, S. 183).

Neben dem schon beschriebenen Zusatz von CO zur Steigerung der Selektivität ist weiter vorgeschlagen worden, die Katalysatoren mit Schwefelverbindungen zu modifizieren. So kann man beispielsweise gemäß FR 1 240 175 durch Behandeln mit Thioethern Katalysatoren erhalten, die selektiv auf die Hydrierung von Acetylen wirken. Schließlich ist aus FR 2 355 792 bekannt, daß sich mit Schwefelwasserstoff dotierte Katalysatoren für die selektive Hydrierung von Butadien eignen. Sie katalysieren jedoch gleichzeitig die Isomerisierung von z.B. Buten-1 zu Buten-2.

Die vorgenannten Verfahren sind unbefriedigend, weil sie entweder auf niedrige Butadiengehalte im Einsatzprodukt beschränkt sind und/oder den Zusatz von CO als selektivitätssteigenden Zusatz erfordern.

Die Verwendung von selektivitätssteigernden Zusätzen, wie CO, ist ungünstig für die Hydrierung butadienreicher Ströme, da hierbei deutlich erhöhte Reaktionstemperaturen benötigt werden, um zu den gewünschten Umsätzen zu gelangen. Hohe Temperaturen führen aber zu verstärkten Nebenreaktionen (Oligomerisation, Polymerisation, Weiterreaktion zum Butan) und verkürzen die Lebensdauer der Katalysatoren.

Damit stellte sich die Aufgabe, ein Verfahren zur selektiven Hydrierung butadienreicher $C_4$-Schnitte vorzuschlagen, das

- an fest angeordneten Katalysatoren abläuft,

- bei dem die zu hydrierenden Verbindungen in einfache ungesättigte Verbindungen übergehen und als solche weitestgehend erhalten bleiben, und

- ohne Zusatz von CO auskommt.

Diese Aufgabe wurde erfindungsgemäß gelöst durch ein Verfahren zur selektiven Hydrierung von Butadien zu Butenen in der Flüssig- oder Rieselphase an fest angeordneten Trägerkatalysatoren, das dadurch gekennzeichnet ist, daß man einen butadienreichen $C_4$-Strom, mit Butadiengehalten von 20 bis 80 Gew.-%, bezogen auf den $C_4$-Strom, in zwei hintereinandergeschalteten Reaktionszonen so hydriert, daß das Hydrierprodukt der ersten Reaktionszone 0,1 bis 20 Gew.-% und das Hydrierprodukt der zweiten Reaktionszone 0,005 bis 1 Gew.-% Restbutadien, bezogen auf den $C_4$-Strom, enthält mit der Maßgabe, daß der Butadiengehalt des Hydrierprodukts der zweiten Reaktionszone mindestens um den Faktor 5 gegenüber dem Butadiengehalt des Hydrierprodukts der ersten Reaktionszone verringert ist, und wobei jede der beiden Reaktionszonen bei Reaktionstemperaturen von 40 bis 120°C, bei Drucken von 5 bis 50 bar und einer auf den $C_4$-Einsatz bezogenen flüssigen Raumzeitgeschwindigkeit von 0,1 bis 30 $h^{-1}$ betrieben wird und daß die Wärmeabfuhr durch Flüssig-Recycle von hydriertem Produkt geregelt wird.

Nach einer bevorzugten Ausführung wird der Butadiengehalt des Hydrierprodukts der zweiten Reaktionszone um mehr als den Faktor 10 und besonders bevorzugt um mehr als den Faktor 20 gegenüber dem Butadiengehalt des Hydrierprodukts der ersten Reaktionszone verringert.

Für das erfindungsgemäße Verfahren können die verschiedensten Butadien enthaltenden $C_4$-Ströme eingesetzt werden. Vorzugsweise werden $C_4$-Ströme eingesetzt, die 30 bis 60 Gew.-% Butadien enthalten. Als weitere Komponenten können Vinylacetylen, Butin-1, Butin-2, Buten-1, Buten-2, i-Buten, n-Butan und/oder i-Butan enthalten sein. Vinylacetylen und Butine werden ebenfalls selektiv zu Butenen hydriert. i-Buten, n-Butan und i-Butan gehen unverändert aus dem erfindungsgemäßen Verfahren hervor.

Das erfindungsgemäße Verfahren wird zweckmäßig in der Flüssig- oder Rieselphase durchgeführt, wobei der Wasserstoff in an sich bekannter Weise in dem flüssigen $C_4$-Strom fein verteilt wird. Vorzugsweise führt man die selektive Hydrierung des Butadiens in der Rieselphase mit fest angeordneten Hydrierkatalysatoren durch.

Als Hydrierkatalysatoren können Edelmetall-Träger-Katalysatoren eingesetzt werden, die Palladium, Platin, Silber, Gold, Rhodium, Ruthenium, Osmium oder Gemische dieser Metalle enthalten. Als Katalysatoren sind Palladium enthaltende Trägerkatalysatoren besonders bevorzugt.

Die beiden Reaktionszonen müssen so voneinander getrennt sein, daß dazwischen Wasserstoff eindosiert und fein verteilt werden kann. Vorzugsweise sind die beiden Reaktionszonen in Form von getrennten Hydrierreaktoren ausgeführt. Der Wasserstoff wird jeweils in der einfachen bis zweifachen der zum berechneten Umsatz stöchiometrisch notwendigen Menge zugegeben, vorzugsweise wird die stöchiometrisch erforderliche Menge bis zu einem Wasserstoffüberschuß in der 1,2fachen Menge zugegeben.

Der für die Hydrierung verwendete Wasserstoff kann bis zu 30 Mol-% an Inertgas, z.B. Methan, enthalten, ohne daß hierdurch die Hydrierung beeinträchtigt wird. Der für das erfindungsgemäße Verfahren verwendete Wasserstoff sollte bevorzugt CO-frei sein; geringe Mengen an CO stören aber nicht.

Die Reaktionsbedingungen in jeder der beiden Reaktionen können in weiten Bereichen variiert werden. So läuft das erfindungsgemäße Verfahren bei Reaktionstemperaturen von 20 bis 200°C, vorzugsweise 40 bis 120°C, bei Drucken von 5 bis 50 bar, vorzugsweise 5 bis 30 bar und einer auf den $C_4$-Einsatz bezogenen flüssigen Raumzeitgeschwindigkeit "liquid hourly space velocity" (lhsv) von 0,1 bis 30 $h^{-1}$, vorzugsweise 1 bis 10 $h^{-1}$ ab.

Das erfindungsgemäße Verfahren weist eine Reihe von Vorteilen auf. Das im Einsatzstoff enthaltene Butadien wird praktisch quantitativ mit sehr hoher Selektivität hydriert. Man erreicht hierbei trotz der sehr hohen Butadienkonzentration eine Butenselektivität "S" von mindestens 96 %.

$$'S'_{Buten} = \frac{\text{Umsatz zu Butenen}}{\text{Gesamt-Umsatz an Butadien}} \times 100$$

Die Hydrierung ist über einen sehr großen Bereich bis hin zu extrem hohen Butadiengehalten selektiv. Die Isomerisierung von Buten-1 zu Buten-2 ist gering und Isobuten wird nicht zu Isobutan umgesetzt. An den Wasserstoff werden keine besonderen Reinheitsanforderungen gestellt, solange keine irreversiblen Katalysatorgifte, wie Blei oder Arsen, enthalten sind. Die Wasserstoffdosierung kann mit automatisch arbeitenden Analysenverfahren leicht geregelt werden.

Da die Selektivität auch bei höherer Reaktionstemperatur erhalten bleibt, werden keine aufwendigen Kühlvorrichtungen oder Anlagen zur Kälteerzeugung benötigt. Die Wärmeabfuhr wird einfach über eine ausreichende Menge an Flüssig-Recycle von hydriertem Produkt geregelt.

Ferner werden bei dem erfindungsgemäßen Verfahren keine merklichen Mengen an Oligomerisationsprodukten gebildet.

Die folgenden Beispiele erläutern das erfindungsgemäße Verfahren, ohne es in irgendeiner Weise zu beschränken.

Beispiel 1

Die Hydrierung eines $C_4$-Schnitts mit der in Tabelle 2 angegebenen Zusammensetzung wurde in zwei Festbettreaktoren durchgeführt, die in Serie gefahren wurden. Beide Reaktoren waren jeweils mit einem Abscheider und einem Flüssigkeitskreislauf ausgestattet. Die jeweiligen Kreislauf- und Abgasmengen konnten über einen großen Bereich variiert werden. Die Reaktion wurde in Rieselfahrweise an einem Trägerkatalysator durchgeführt, der 0,5 Gew.-% Palladium enthielt. Das verwendete Hydriergas bestand aus 90 Vol.% Wasserstoff und 10 Vol.% Methan und wurde den beiden Reaktoren in bezug auf den Butadiengehalt des Einsatz-$C_4$-Stroms mengengeregelt zudosiert. Hinter jeder der einzelnen Hydrierzonen bestand die Möglichkeit der Probenahme. Die auf den $C_4$-Einsatz bezogene flüssige Raum-Zeit-Geschwindigkeit (lhsv) betrug 8,8 l/l x h.

Die erste Stufe der Hydrierung wurde bei einer Reaktoreintrittstemperatur von 53°C und einem Reaktordruck von 14,1 bar durchgeführt.

Die zweite Stufe der Hydrierung fand bei einer Reaktoreintrittstemperatur von 63°C und einem Gesamtdruck im Reaktor von 9,5 bar statt.

Über beide Stufen wurde der Butadiengehalt von 46,1 Gew.-% auf 0,2 Gew.-% gesenkt bei einer Butenselektivität von 97,6 %.

Die erhaltenen Produktzusammensetzungen in Gewichtsprozenten sind in Tabelle 2 dargestellt.

Tabelle 2

|  | $C_4$-Einsatz | Hydrierprodukt nach der 1. Stufe | Hydrierprodukt nach der 2. Stufe |
|---|---|---|---|
| Butadien | 46,1 | 5,8 | 0,2 |
| Buten-1 | 11,5 | 35,8 | 32,6 |
| trans-Buten-2 | 3,8 | 17,9 | 25,0 |
| cis-Buten-2 | 2,6 | 3,8 | 5,1 |
| i-Buten | 23,9 | 23,9 | 23,9 |
| i-Butan | 4,3 | 4,3 | 4,3 |
| n-Butan | 7,8 | 8,5 | 8,9 |

Beispiel 2
(Vergleichsbeispiel)

Innerhalb einer Hydrierzone wurde in Rieselfahrweise bei Einsatz des gleichen $C_4$-Schnitts und Katalysators wie in Beispiel 1 der Butadiengehalt auf 0,3 Gew.-% gesenkt, wobei die Zusammensetzung des Hydriergases unverändert blieb.

Die Reaktion wurde bei einer auf den $C_4$-Einsatz bezogenen flüssige Raum-Zeit-Geschwindigkeit (lhsv) von 5,4 l/l x h durchgeführt, wobei die Reaktoreintrittstemperatur 67°C und der Gesamtdruck im Reaktor 19,8 bar betrugen.

Bei dieser einstufigen Hydrierung eines butadienreichen $C_4$-Schnitts wurde nur eine Butenselektivität von 71,4 % erzielt.

Das Ergebnis dieses Versuchs ist in Tabelle 3 zusammengefaßt, wobei die Zusammensetzung in Gewichtsprozenten angegeben wird.

Tabelle 3

|  | $C_4$-Einsatz | Hydrierprodukt bei einstufiger Fahrweise |
|---|---|---|
| Butadien | 46,1 | 0,3 |
| Buten-1 | 11,5 | 8,0 |
| trans-Buten-2 | 3,8 | 28,8 |
| cis-Buten-2 | 2,6 | 13,8 |
| i-Buten | 23,9 | 23,9 |
| i-Butan | 4,3 | 4,3 |
| n-Butan | 7,8 | 20,9 |

Beispiel 3

Mittels der gleichen Versuchsanordnung wie in Beispiel 1 wurde ein butadienreicher $C_4$-Schnitt mit der in Tabelle 4 angegebenen Zusammensetzung in einer zweistufigen Fahrweise selektiv hydriert. Dabei wurde der gleiche Pd-haltige Trägerkatalysator verwendet; die Hydriergaszusammensetzung betrug 90 Vol.% Wasserstoff und 10 Vol.% Methan. Die eingestellte auf den $C_4$-Einsatz bezogene flüssige Raum-Zeit-Geschwindigkeit (lhsv) betrug 6,0 l/l x h.

Die erste Stufe der Hydrierung wurde bei einer Reaktoreintrittstemperatur von 55°C und einem Reaktordruck von 20,2 bar und die zweite Stufe bei einer Reaktoreintrittstemperatur von 66°C und einem Reaktordruck von 10,4 bar durchgeführt.

Der Butadiengehalt wurde über beide Stufen von 71,1 Gew.-% auf 0,05 Gew.-% gesenkt bei einer Gesamtselektivität zu den Butenen von 97,0 %.

Die erhaltenen Produktzusammensetzungen in Gewichtsprozenten sind in Tabelle 4 dargestellt.

Tabelle 4

|  | $C_4$-Einsatz | Hydrierprodukt nach der 1. Stufe | Hydrierprodukt nach der 2. Stufe |
|---|---|---|---|
| Butadien | 71,1 | 4,1 | 0,05 |
| Buten-1 | 7,3 | 38,4 | 32,5 |
| trans-Buten-2 | 2,9 | 29,7 | 30,7 |
| cis-Buten-2 | 1,8 | 9,5 | 17,7 |
| i-Buten | 15,0 | 15,0 | 15,0 |
| i-Butan | 0,4 | 0,4 | 0,4 |
| n-Butan | 1,5 | 2,9 | 3,6 |

**Patentansprüche**

1. Verfahren zur selektiven Hydrierung von Butadien zu Butenen in der Flüssig- oder Rieselphase an fest angeordneten Edelmetall-Träger-Katalysatoren, dadurch gekennzeichnet, daß man einen butadienreichen $C_4$-Strom, mit Butadiengehalten von 20 bis 80 Gew.-%, bezogen auf den $C_4$-Strom, in zwei hintereinandergeschalteten Reaktionszonen so hydriert, daß das Hydrierprodukt der ersten Reaktionszone 0,1 bis 20 Gew.-% und das Hydrierprodukt der zweiten Reaktionszone 0,005 bis 1 Gew.-% Restbutadien, bezogen auf den $C_4$-Strom, enthält mit der Maßgabe, daß der Butadiengehalt des Hydrierprodukts der zweiten Reaktionszone mindestens um den Faktor 5 gegenüber dem Butadiengehalt des Hydrierprodukts der ersten Reaktionszone verringert ist, und wobei jede der beiden Reaktionszonen bei Reaktionstemperaturen von 40 bis 120°C, bei Drucken von 5 bis 50 bar und einer auf den $C_4$-Einsatz bezogenen flüssigen Raumzeitgeschwindigkeit von 0,1 bis 30 $h^{-1}$ betrieben wird und daß die Wärmeabfuhr durch Flüssig-Recycle von hydriertem Produkt geregelt wird.

2. Verfahren nach Anspruch 1, dadurch gekennzeichnet, daß die zwei Reaktionszonen in Form von getrennten Hydrierreaktoren ausgebildet sind.

3. Verfahren nach Anspruch 1 oder 2, dadurch gekennzeichnet, daß der Wasserstoff jeder der beiden Reaktionszonen jeweils in der einfachen bis zweifachen der stöchiometrisch notwendigen Menge zugegeben wird.

4. Verfahren nach den Ansprüchen 1 bis 3, dadurch gekennzeichnet, daß nach der ersten Reaktionszone ein Restbutadiengehalt von 0,1 bis 10 Gew.-%, eingestellt wird.

5. Verfahren nach den Ansprüchen 1 bis 4, dadurch gekennzeichnet, daß als Katalysator Palladium auf inerten Trägern verwendet wird.

6. Verfahren nach den Ansprüchen 1 bis 5, dadurch gekennzeichnet, daß zur Hydrierung wasserstoffhaltige Gase mit Inertgasgehalten bis zu 30 Mol.-% verwendet werden.

**Claims**

1. A process for selective hydrogenation of butadiene to butenes in the liquid or trickle phase over fixed-bed supported noble metal catalysts, characterized in that a butadiene-rich $C_4$ stream, having butadiene contents from 20 to 80 % by weight, based on the $C_4$ stream, is hydrogenated in two successive reaction zones in such a way that the hydrogenation product of the first reaction zone comprises from 0.1 to 20 % by weight of residual butadiene, based on the $C_4$ stream, and the hydrogenation product of the second reaction zone comprises from 0.005 to 1 % by weight of residual butadiene, based on the $C_4$ stream, with the proviso that the butadiene content of the hydrogenation product of the second reaction zone is at least 5 times lower than the butadiene content of the hydrogenation product of the first reaction zone, each of the two reaction zones being operated at reaction temperatures from 40 to 120°C, at pressures from 5 to 50 bar and at a liquid space-time velocity, based on the $C_4$ feed, of from 0.1 to 30 h$^{-1}$, and in that the heat removal is regulated through a liquid recycle of hydrogenated product.

2. A process as claimed in claim 1, characterized in that the two reaction zones take the form of separate hydrogenation reactors.

3. A process as claimed in claim 1 or 2, characterized in that the hydrogen is added to each of the two reaction zones in one to two times the stoichiometrically necessary amount in each case.

4. A process as claimed in any of claims 1 to 3, characterized in that a residual butadiene content from 0.1 to 10 % by weight is set downstream of the first reaction zone.

5. A process as claimed in any of claims 1 to 4, characterized in that the catalyst used is palladium on inert supports.

6. A process as claimed in any of claims 1 to 5, characterized in that hydrogenation is effected using hydrogen-comprising gases having inert gas contents of up to 30 mol%.

**Revendications**

1. Procédé d'hydrogénation sélective de butadiène en butènes en phase liquide ou ruisselante en présence de catalyseurs sur support à base de métaux précieux disposés en lit fixe, caractérisé en ce que l'on hydrogène un courant d'hydrocarbures en $C_4$ riche en butadiène, ayant des teneurs en butadiène de 20 à 80% en poids par rapport au courant d'hydrocarbures en $C_4$, dans deux zones de réaction successives, de telle sorte que le produit d'hydrogénation de la première zone de réaction contienne de 0,1 à 20% en poids de butadiène résiduel par rapport au courant d'hydrocarbures en $C_4$ et que le produit d'hydrogénation de la seconde zone de réaction en contienne de 0,005 à 1% en poids, étant spécifié que la teneur en butadiène du produit d'hydrogénation de la seconde zone de réaction est abaissée d'un facteur au moins égal à 5 par rapport à la teneur en butadiène du produit d'hydrogénation de la première zone de réaction, chacune des deux zones de réaction étant exploitée à une température de réaction de 40 à 120°C, sous une pression de 5 à 50 bar et à une vitesse espace-temps liquide, rapportée à l'introduction d'hydrocarbures en $C_4$, de 0,1 à 30 h$^{-1}$, et en ce que l'élimination de chaleur est réglée par recyclage à l'état liquide de produit hydrogéné.

2. Procédé selon la revendication 1, caractérisé en ce que les deux zones de réaction sont réalisées sous forme de réacteurs d'hydrogénation séparés.

3. Procédé selon la revendication 1 ou 2, caractérisé en ce que l'hydrogène est fourni à chacune des deux zones de réaction à raison d'une fois à deux fois la quantité stoechiométriquement nécessaire.

4. Procédé selon l'une quelconque des revendications 1 à 3, caractérisé en ce qu'à la suite de la première zone de réaction, une teneur en butadiène résiduel de 0,1 à 10% en poids est réglée.

5. Procédé selon l'une quelconque des revendications 1 à 4, caractérisé en ce que du palladium sur support inerte est utilisé comme catalyseur.

6. Procédé selon l'une quelconque des revendications 1 à 5, caractérisé en ce que des gaz contenant de l'hydrogène, ayant des teneurs en gaz inertes de 30% en moles au maximum, sont utilisés pour l'hydrogénation.